# EUROPÄISCHE PATENTSCHRIFT

(11) **EP 2 338 423 B1**
(45) Veröffentlichungstag und Bekanntmachung des Hinweises auf die Patenterteilung: **18.10.2017**
(21) Anmeldenummer: 10187986.4
(22) Anmeldetag: 19.10.2010
(51) Int. Cl.: A61B 17/3203, B26F 3/00, F04B 49/02

(54) **Verfahren zur Förderung eines Fluids sowie Vorrichtung zur Erzeugung eines Volumenstromes**
Method for conveying a fluid and device for producing a volume flow
Procédé pour transporter un fluide et dispositif pour produire un débit volumétrique

(30) Priorität: 23.12.2009 DE 102009055227
(43) Veröffentlichungstag der Anmeldung: 29.06.2011
(73) Patentinhaber: Human Med AG, 19061 Schwerin (DE)
(72) Erfinder: Kensy, Arnd, 14552 Michendorf - OT Wilhelmshorst (DE); Winkler, Konrad-Wenzel, 19417 Warin (DE); Runow, Andreas, 19055 Schwerin (DE); Niklas, Frank, 19386 Lübz (DE); Schlee, Gernot, 19057 Schwerin (DE)
(74) Vertreter: Gulde & Partner

(56) Entgegenhaltungen:
- WO-A1-2005/034777
- WO-A2-01/97700
- WO-A2-2004/112623
- DE-T2- 69 927 153
- US-A- 930 989
- US-A- 3 520 477
- US-A- 5 591 184
- US-A- 5 735 815
- US-A1- 2002 116 021

## Beschreibung

Die vorliegende Erfindung betrifft ein Verfahren zur Förderung eines Fluids zwecks Erzeugung eines Fluid-Strahles, bei dem das Fluid von einer Pumpe druckbeaufschlagt mit einem bestimmten Volumenstrom einer Austrittsöffnung zugeleitet wird.

Außerdem betrifft die vorliegende Erfindung eine Vorrichtung zur Erzeugung eines Volumenstromes zwecks Realisierung eines Fluid-Strahles zum Schneiden von festem oder pastösem Material, welche eine Pumpe umfasst, mittels derer eine Betriebsweise zur Druckerzeugung im Fluid und eine Betriebsweise zur Saugwirkungserzeugung realisierbar ist.

Es ist bekannt, mittels Vorrichtungen zum Wasserstrahlen biologische Struktur zu schneiden und gleichzeitig oder zeitnah versetzt abzusaugen. Insbesondere werden derartige Vorrichtungen zur Fettabsaugung verwendet. Derartige Vorrichtungen umfassen im Wesentlichen ein Fluid-Reservoir sowie eine Pumpe, die das Fluid druckbeaufschlagt zu einem Werkzeug, welches zur Strahlerzeugung dient, leitet, wobei das Fluid druckbeaufschlagt in einem Strahl auf oder in einen festen Körper oder weiches Gewebe geleitet wird und somit Segmente dieses Körpers/Gewebes abtrennt. Durch Erzeugung eines Unterdrucks in der Nähe der Strahldüse werden die abgetrennten Segmente in das Werkzeug hineingesaugt und einem Sammelbehälter zugeführt. Es ist somit minimalinvasiv auch die Absaugung bzw. Entfernung von biologisch gewachsener Struktur realisierbar. Die Werkzeuge bzw. Vorrichtungen zur Durchführung des Strahlschneidens sowie des Absaugens sind aus hygienischen Gründen oftmals als Einweg-Geräte konzipiert. Außerdem müssen die Werkzeuge bzw. Vorrichtungen zwecks einfacher manueller Handhabung ein geringes Gewicht aufweisen. Die gesamte Vorrichtung muss selbsterklärend bedienbar sowie ständig einsetzbar sein.

Aus der EP 1 670 371 B1 ist eine Fördereinrichtung für Sterilmedien bekannt, die zur Druckerzeugung zwei Kolben-Zylinder-Einheiten aufweist, deren Ansaug- und Drucktakte zur Druckerzeugung und gleichzeitigen Saugwirkungserzeugung einander überlappen, so dass der aus der Düse austretende Volumenstrom ständig aufrecht erhalten werden kann. Nachteilig an dieser Fördereinrichtung ist jedoch der relativ hohe konstruktive Aufwand zur Realisierung des bedarfsgerechten Volumenstroms.

Aus der WO 2004/112623 A2 ist eine Wasserstrahleinrichtung zum Trennen einer biologischen Struktur bekannt, die mit einer Pumpe zur Druckerzeugung und Saugwirkungserzeugung ausgestattet ist, die lediglich eine Kolben-Zylinder-Einheit aufweist. Der Kolben der Pumpe wird mittels eines Exzenters angetrieben, wobei bei Kontaktierung des Kolbens mit dem Exzenter am maximalen Exzenterradius der Kolben sich in seinem vorderen Totpunkt und damit am Ende der Bewegung zur Druckerzeugung befindet, und bei Kontaktierung des Kolbens mit dem Exzenter am minimalen Radius sich der Kolben am hinteren Totpunkt befindet und somit am Ende des Weges zur Erzeugung der Saugwirkung. Je nach Bewegungsrichtung des Exzenters lässt sich somit eine Betriebsweise zur Druckerzeugung oder eine Betriebsweise zur Saugwirkungserzeugung realisieren.

Beim Betrieb der Wasserstrahleinrichtung, insbesondere beim Trennen einer biologischen Struktur, muss der Operateur ggf. den Strahl unterbrechen, um das Werkzeug neu zu positionieren und/oder operative Zwischenschritte durchzuführen und/oder optisch das Operationsgebiet zu überprüfen. Diese Unterbrechung wird üblicherweise durch die Betätigung einer Betätigungseinrichtung erreicht. Nach Beendigung der Unterbrechung soll der Volumenstrom mit vorher eingestellten Parametern wieder auf das zu schneidende Material gerichtet werden.

Bei der Wasserstrahleinrichtung gemäß der WO 2004/112623 A2 besteht bei Unterbrechung und Fortsetzung des Wasserstrahlens das Problem, dass bei Unterbrechung des Fluid-Strahles der Exzenter in einer bestimmten Winkelposition angehalten wird und bei Fortsetzung des Strahlvorganges der Exzenter wieder in Rotation versetzt wird, so dass er den Kolben weiterhin nach vorne zur Druckerzeugung bewegt. Es steht dem Operateur für den Strahlvorgang somit lediglich das Volumen an Fluid zur Verfügung, welches im Zylinder der Kolben-Zylinder-Einheit je Hub des Kolbens aufgenommen werden kann. Bei länger andauernden Operationen bzw. größerem Fluid-Bedarf muss demzufolge der Strahlvorgang unterbrochen werden, so dass der Kolben bei weiterer Rotation des Exzenters und gleichzeitiger Erzeugung einer Saugwirkung Richtung Vorratsbehälter wieder in den hinteren Totpunkt hochgefahren werden kann, so dass der Zylinder-Raum erneut mit Fluid gefüllt wird und anschließend bei weiterer oder gegenläufiger Rotation des Exzenters wieder der Druck im Fluid zwecks Strahlerzeugung aufgebaut werden kann.

Aus WO 01/97700 ist eine Einrichtung zur Erzeugung eines Flüssigkeitsstrahles für das Entfernen insbesondere von biologischem Gewebe sowie eine Verwendung der Einrichtung bekannt. Hierbei kann durch eine Bedienperson eine flüssigkeitsstrahlerzeugende Pumpe abgeschaltet werden, so dass diese einen drucklosen Zustand annimmt.

US 5,591,184 A offenbart ein chirurgisches Instrument, bei dem ein unter geringem Druck stehendes Fluid mittels eines Gases pulsförmig zu einer Austrittsöffnung des chirurgischen Instrumentes gefördert werden kann.

Der vorliegenden Erfindung liegt somit die Aufgabe zugrunde, ein Verfahren und eine Vorrichtung zur Verfügung zu stellen, mittels derer in einfacher und kostengünstiger konstruktiver Ausführungsform ein bestimmter Fluid-Volumenstrom bei Operationen mit unterschiedlichen Volumenstromanforderungen über einen möglichst langen Zeitraum zur Verfügung gestellt werden kann.

Die vorliegende Erfindung wird durch das Verfahren zur Förderung eines Fluids nach Anspruch 1 sowie durch die Vorrichtung zur Erzeugung eines Volumenstroms nach Anspruch 6 erfindungsgemäß gelöst. Vorteilhafte Ausgestaltungen des Verfahrens sind in den Unteransprüchen 2 bis 5 angegeben. Vorteilhafte Ausgestaltungen der erfindungsgemäßen Vorrichtung sind in den Unteransprüchen 7 bis 13 angegeben.

Erfindungemäß wird ein Verfahren zur Förderung eines Fluids zwecks Erzeugung eines Fluid-Strahles zur Verfügung gestellt, bei dem das Fluid von einer Pumpe druckbeaufschlagt mit einem bestimmten Volumenstrom einer Austrittsöffnung zugeleitet wird, wobei bei einer Unterbrechung des Volumenstroms die Pumpe automatisch aus einer Betriebsweise zur Druckerzeugung in eine Betriebsweise zur Saugwirkungserzeugung zwecks Ansaugung von Fluid umgeschaltet wird, wobei der Saugbetrieb so lange realisiert wird, bis ein Signal über die Beendigung der Unterbrechung des Volumenstromes generiert wird, woraufhin die Pumpe in die Betriebsweise zur Druckerzeugung umschaltet. Das Verfahren kann dazu dienen, das Fluid in ein chirurgisches oder medizinisches Instrument einzuspeisen, dass heißt in ein Gerät zur Flüssigkeitsstrahlung zwecks Schneiden bzw. Freispülung von biologischer Struktur, wie z. B. Fett oder Gewebe, welches danach oder gleichzeitig abgesaugt werden kann. Das Verfahren kann somit zur Gewichtsreduzierung und/oder zur Reduzierung des Körpervolumens eines Patienten eingesetzt werden.

Alternativ kann jedoch auch vorgesehen sein, dass das Verfahren nicht bei einer medizinischen oder chirurgischen Behandlung eines menschlichen oder tierischen Körpers eingesetzt wird, sondern z. B. zur Säuberung oder Entfernung von Strukturen, ggf. biologischen Strukturen, von Oberflächen.

Das erfindungsgemäße Verfahren ist derart eingerichtet, dass sehr einfach und kostengünstig aufgebaute Pumpen eingesetzt werden können, wobei diese Pumpen entweder nur im Druckbetrieb oder nur im Saugbetrieb betrieben werden können und eingerichtet sind, zwischen dem Druck- und Saugbetrieb umgeschaltet zu werden. Da die Pumpe in einem Einweg-Gerät einen relativ großen Kostenfaktor bedingt, lassen sich durch einfache Pumpen die Herstellungskosten stark minimieren. Trotz des einfachen und kostengünstigen Aufbaus lässt sich mit der Pumpe das Fluid, welches in den meisten Anwendungen eine Flüssigkeit ist, mit einem Volumenstrom bestimmter Größe zuverlässig der Strahldüse zuführen. Dabei ist unter einer Unterbrechung des Volumenstroms ein Absinken der physikalischen Größe Volumenstrom in einem bestimmten Zeitfenster unter einen bestimmten Minimalwert zu verstehen. Bei der Unterbrechung des Volumenstroms wird ein diesbezügliches Signal von einer Betätigungseinrichtung zur Beeinflussung des eingestellten Volumenstroms und/oder von einem Sensor generiert. Der Sensor kann in der Leitung zur Auslassöffnung, an der Auslassöffnung selbst oder in der Antriebs-Einheit angeordnet sein. Der Sensor an der Leitung oder an der Auslassöffnung kann das Vorhandensein oder Nichtvorhandensein eines Volumenstroms detektieren. Auch kann ein Sensor eines Wegmesssystems eines linearen Stellzylinders, der die Kolben-Zylinder-Einheit bewegt, die Endposition des Kolbens in seinem Weg zur Druckerzeugung detektieren. Das von dem jeweiligen Sensor oder dem Wegmesssystem generierte Signal wird entweder der Pumpe direkt eingegeben, woraufhin die Pumpe in die Betriebsweise zur Saugwirkungserzeugung umschaltet, oder das generierte Signal wird einer Steuerungseinrichtung zugeleitet, die ein entsprechendes, die Umschaltung bewirkendes Signal der Pumpe zuleitet.

In besonderer Ausgestaltung der Erfindung kann der Volumenstrom der Austrittsöffnung pulsierend zugeleitet werden, wobei die Zeitintervalle zwischen den einzelnen, die Pulsierung realisierenden Volumenstrom-Abschnitten zwischen 0,05 und 30 Sekunden betragen.

Das heißt, dass der eingestellte Soll-Volumenstrom Intervalle umfasst, wobei ein intervallbedingtes Aussetzen des Volumenstroms keine Unterbrechung im Sinne der Erfindung darstellt. Das heißt, dass die Pumpe intervallunabhängig bei Unterbrechung der Betriebsweise zur Druckerzeugung auf die Betriebsweise zur Erzeugung der Saugwirkung umschaltet. Je nach Einstellung des Volumenstroms ist somit ein bestimmter Soll-Fluidstrom vorgegeben, der unterschiedliche Parameter hinsichtlich des Druckes, der Frequenz und anderer physikalischer Werte haben kann, wobei eine Absenkung des physikalischen Volumenstromwertes über eine bestimmte Zeitspanne unter einen bestimmten Grenzwert als Unterbrechung des Fluid-Stromes gewertet wird und die Pumpe erfindungsgemäß umschaltet. Bei Anwendung einer Steuerungseinrichtung sollen dabei die Pulsfrequenzen, Intervalle und Zeitdauer der Betriebsweisen zur Druckerzeugung sowie zur Ansaugwirkungserzeugung mittels der Steuerungseinrichtung im Wesentlichen frei einstellbar und programmierbar sein. Sind die Zeitintervalle zwischen den Volumenstromabschnitten Null oder nahe Null, kann ein pulsationsfreier oder im Wesentlichen pulsationsfreier Volumenstrom erzeugt werden. Erfindungsgemäß ist das Verfahren derart ausgestaltet, dass nach erfolgter Umschaltung der Saugbetrieb so lange realisiert wird, bis ein Signal über die Beendigung der Unterbrechung des Volumenstroms generiert wird, woraufhin die Pumpe in die Betriebsweise zur Druckerzeugung zurückschaltet. Dieses Signal über die Beendigung der Unterbrechung des Volumenstroms kann von einer Betätigungseinrichtung, die zuvor zur Unterbrechung des Volumenstromes diente, erzeugt werden oder durch einen Sensor zur Erfassung der Erschöpfung des Fassungsvermögens der Pumpe in die Betriebsweise zur Saugwirkungserzeugung oder durch einen, das gesamte Verfahren beendenden, Ausschaltbefehl generiert werden.

Zwecks Anpassung des erfindungsgemäßen Verfahrens an den voraussichtlichen Fluid-Bedarf des Benutzers ist die Zeitdauer der Betriebsweise zur Druckerzeugung und/oder die Zeitdauer der Betriebsweise zur Saugwirkungserzeugung mittels einer Steuerungseinrichtung vor oder während des Verfahrens eingestellt. Es lässt sich somit variabel die Hublänge des Kolbens steuern und somit Einfluss nehmen auf das in einem Zeitfenster unter einem bestimmten Druck zur Verfügung stehende Fluid-Volumen, insbesondere bei gleich bleibender Leistung eines motorischen Antriebes der Pumpe.

Vorteilhafterweise ist dabei vorgesehen, dass die Zeitdauer für die Betriebsweise zur Saugwirkungserzeugung kürzer ist als die Zeitdauer für die Betriebsweise zur Druckerzeugung. Damit ist es möglich, dem Benutzer in kürzerer Zeitspanne als der Strahldauer wieder das maximale Fluid-Volumen zur Verfügung zu stellen.

Bei Anwendung einer Kolbenpumpe kann eine Steuerungseinrichtung den Kolbenweg derart steuern, dass der Kolbenweg bei der Betriebsweise zur Saugwirkungserzeugung kürzer, gleich oder länger ist als der in der zuvor realisierten Betriebsweise zur Druckerzeugung zurückgelegte Kolbenweg. Diese Verfahrenswegausbildung kann jedoch nur in einem einzelnen Druck-Saug-Zyklus durchgeführt werden.

Mittels des erfindungsgemäßen Verfahrens wird sichergestellt, dass bei Unterbrechung des Volumenstroms das Zeitfenster der Unterbrechung genutzt wird, um den in der Kolben-Zylinder-Einheit zur Verfügung stehenden Raum durch die Umschaltung der Pumpe in die Betriebsweise zur Saugwirkungserzeugung erneut mit Fluid zu befüllen, so dass nach Beendigung der Unterbrechung das maximale Fluid-Volumen erneut zur Verfügung steht.

Das Verfahren wird unabhängig von der jeweiligen Kolbenposition durchgeführt. Da üblicherweise beim Strahlschneiden, insbesondere bei medizinischen oder chirurgischen Eingriffen, der Operateur zwecks Begutachtung des bisherigen Strahlergebnisses den Strahlvorgang unterbricht, ist erfindungsgemäß sichergestellt, dass durch die in der Unterbrechungsphase realisierte Saugwirkung über einen sehr langen und lediglich unterbrochenen Zeitraum Fluid zum Strahlen zur Verfügung steht.

Zur Durchführung des Verfahrens wird erfindungsgemäß eine Vorrichtung zur Erzeugung eines Volumenstroms zwecks Realisierung eines Fluid-Strahles zum Schneiden oder Freispülen von festem Material oder weichem Gewebe zur Verfügung gestellt, wobei die Vorrichtung eine Pumpe umfasst, mittels derer eine Betriebsweise zur Druckerzeugung im Fluid und eine Betriebsweise zur Saugwirkungserzeugung realisierbar ist. Erfindungsgemäß ist die Vorrichtung derart ausgestaltet, dass bei Unterbrechung des eingestellten Volumenstromes die Pumpe automatisch aus der Betriebsweise zur Druckerzeugung in eine Betriebsweise zur Saugwirkungserzeugung zwecks Ansaugung von Fluid umschaltbar ist bzw. umschaltet. Dabei umfasst die Vorrichtung vorteilhafterweise eine Steuerungseinrichtung, die die Umschaltung der Pumpe steuert bzw. auslöst. Das feste oder weiche Material, welches mittels des Fluid-Strahles geschnitten oder freigespült wird, kann dabei biologisches Gewebe (wie z. B. Fett) sein, so dass die Vorrichtung zum Schneiden oder Freispülen und anschließenden oder gleichzeitigen Absaugen von Fett geeignet ist. Mittels der Pumpe der Vorrichtung wird ein Volumenstrom in einer von der Vorrichtung umfassten und der Pumpe in Volumenstromrichtung nachgeschalteten Leitung erzeugt und einer Ausgangsöffnung, z. B. in Form einer Düse, zugeführt. Bei Unterbrechung dieses Volumenstroms in der Leitung wird erfindungsgemäß automatisch die Pumpe in die Betriebsweise zur Saugwirkungserzeugung umgeschaltet.

Zur Vereinfachung der Bedienung der Vorrichtung ist vorgesehen, dass die Vorrichtung eine Betätigungseinrichtung umfasst, mittels derer manuell die Unterbrechung des Volumenstromes realisierbar ist. Unter manueller Betätigung der Betätigungseinrichtung kann dabei auch ein Fußschalter verstanden werden, wobei das wesentliche Merkmal der Betätigungseinrichtung die Möglichkeit der direkten Betätigung durch eine Bedienperson ist.

Dabei kann die Betätigungseinrichtung derart ausgestaltet sein, dass sie bei Betätigung zwecks Unterbrechung des Volumenstroms ein Signal über die Unterbrechung des Volumenstromes generiert. Das heißt, dass durch die Betätigung der Betätigungseinrichtung die Pumpe umgeschaltet werden kann, oder dass durch Betätigung der Betätigungseinrichtung ein Signal über die Unterbrechung des Volumenstroms generiert werden kann, welches einer von der Vorrichtung umfassten Steuerungseinrichtung zugeleitet wird, die die Pumpe derart ansteuert, dass diese umschaltet. In alternativer Ausgestaltung ist vorgesehen, dass die Betätigungseinrichtung zumindest bei erstmaliger Betätigung den Volumenstrom unterbricht und mechanisch gleichzeitig die Pumpe umschaltet. In weiterer Ausgestaltung der Vorrichtung kann diese bei nochmaliger Betätigung der Betätigungseinrichtung ein Zurückschalten der Pumpe in die Betriebsweise zur Druckerzeugung realisieren.

Um die Herstellungskosten der Vorrichtung zu minimieren, ist vorgesehen, dass die Pumpe eine Kolbenpumpe mit nur einer Kolben-Zylinder-Einheit ist. Das heißt, dass die Pumpe nur eine Pumpenkammer aufweist, die einen ausreichend langen statisch konstanten oder schwellenden bzw. pulsierenden Flüssigkeitsstrahl erzeugen kann, wobei die Unterbrechungen der Druckphase in der Anwendung, wie z. B. einer chirurgischen oder medizinischen Operation, genutzt werden, um die Pumpenkammer wieder schnellstmöglich durch Umschaltung auf die Betriebsweise zur Saugwirkungserzeugung mit Fluid zu befüllen.

Zwecks Antrieb des Kolbens der Kolbenpumpe ist vorgesehen, dass dieser mit einem motorisch betriebenen Spindeltrieb oder einem linearen Stellzylinder bewegbar ist. Das heißt, durch Kopplung einer Spindel, die mit einer an eine motorische Antriebseinheit gekoppelten Mutter zusammenwirkt, kann die Spindel und somit auch der Kolben translatorisch bewegt werden. Alternativ ist vorgesehen, dass die Spindel selbst an eine motorische Antriebseinheit gekoppelt ist und die auf der Spindel angeordnete Mutter ihre translatorische Bewegung auf den Kolben überträgt.

In besonderer Ausführungsform ist vorgesehen, dass die Kolbenpumpe motorisch angetrieben ist und die Vorrichtung ein Getriebe umfasst, welches bei gleicher Drehzahl des motorischen Antriebes die Bewegung des Kolbens in der Betriebsweise zur Saugwirkungserzeugung mit größerer Geschwindigkeit als die Bewegung des Kolbens in der Betriebsweise zur Druckerzeugung realisierbar macht. Auch ist bevorzugt vorgesehen, dass der motorische Antrieb so ausgelegt ist, dass er in der Betriebsweise der Saugwirkungserzeugung, wo nur eine geringe Last wirkt, eine entsprechend höhere Drehzahl als in der Betriebsweise der Druckerzeugung mit hoher Last generiert.

Zwecks Feststellung der Unterbrechung des Volumenstroms kann die Vorrichtung einen ersten Sensor in einer in Volumenstromrichtung der Pumpe nachgeschalteten Leitung aufweisen, mittels dem eine Unterbrechung des Volumenstroms erfassbar ist. Alternativ kann der erste Sensor auch außerhalb der Leitung, aber in der Nähe der Ausgangsöffnung, angeordnet sein. Der Sensor kann bei Erfassung der Unterbrechung des Volumenstroms ein Signal über die Unterbrechung generieren, welches der Steuerungseinrichtung zugeleitet wird, die die Pumpe derart ansteuert, dass diese umschaltet.

Alternativ oder hinzukommend kann die Vorrichtung an der Kolbenpumpe oder Antriebseinheit ein Wegmesssystem aufweisen, mittels dem die Positionierung des Kolbens in jeder Lageposition bei der Betriebsweise zur Druckerzeugung und damit die Unterbrechung des Volumenstroms detektierbar ist. Dieses Wegmesssystem kann die Stellung des Kolbens im vorderen Totpunkt detektieren und somit ebenfalls die Unterbrechung des Volumenstroms feststellen und sodann ein Signal über die Unterbrechung des Volumenstroms generieren, welches der von der Vorrichtung umfassten Steuerungseinrichtung zugeleitet wird, die die Pumpe derart ansteuert, dass diese umschaltet. Das heißt, dass bei vollständiger Entleerung der Pumpenkammer zu diesem Zeitpunkt eine Zwangsbefüllung in kürzester Zeit durch Einleitung der Betriebsweise der Saugwirkungserzeugung realisiert wird. (Das Wegmesssystem ist eine Wegerfassung in der Antriebseinheit/Stellzylinder, die über den gesamten Weg die Position der Druckstange=Kolben erkennt).

Vorteilhafterweise umfasst dabei die Vorrichtung die bereits genannte Steuerungseinrichtung, die derart ausgestaltet ist, dass mit ihr die zu erzeugenden Volumenstrom-Intervalle und/oder die Zeitabstände zwischen den Volumenstrom-Intervallen und/oder die Zeitdauer für die beiden Betriebsweisen zum Ansaugen und Druckerzeugen einstellbar sind. Das heißt, dass die Steuerungseinrichtung derart programmierbar ist, dass der durch sie gesteuerte Volumenstrom mit seinen bestimmten Soll-Parametern einstellbar ist. Der eingestellte bzw. einstellbare Volumenstrom kann dabei ein konstanter, ein schwellender oder pulsierender Volumenstrom sein. Außerdem kann die Steuerungseinrichtung dazu dienen, Signale über die Unterbrechung und/oder über die Aufhebung bzw. Beendigung der Unterbrechung der Pumpe zuzuleiten oder entsprechende Steuersignale zu erzeugen und der Pumpe zwecks Umschaltung zuzuleiten. Die erfindungsgemäße Vorrichtung ist somit derart ausgestaltet, dass sie mit geringem konstruktiven und damit fertigungs- sowie materialtechnischem Aufwand und geringem Gewicht dem Nutzer über einen langen, ggf. unterbrochenen Zeitraum ein maximales Fluid-Volumen zur Strahlerzeugung zur Verfügung stellt.

Zur Erläuterung der Erfindung wird diese anhand der beiliegenden Zeichnung beschrieben.

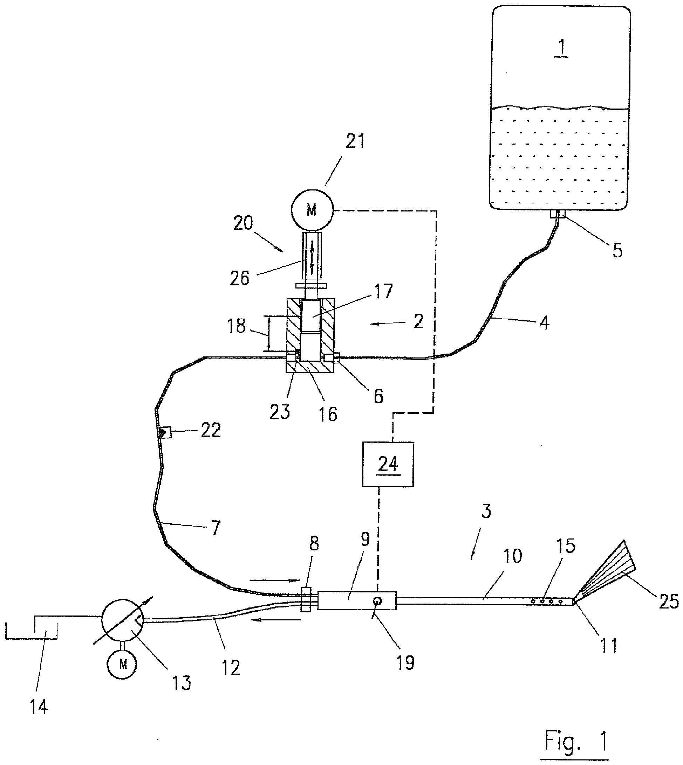

Es zeigt dabei die einzige Figur 1 eine erfindungsgemäße Vorrichtung zur Durchführung des erfindungsgemäßen Verfahrens zur Förderung eines Fluides.

Im Wesentlichen ist die erfindungsgemäße Vorrichtung aus drei Hauptbestandteilen zusammengesetzt, nämlich einem Vorratsbehälter 1, einer Pumpe 2 sowie einem Operationswerkzeug 3. Vom Vorratsbehälter 1 wird Fluid der Pumpe 2 zugeleitet, die das Fluid unter einem erhöhten Druck zum Operationswerkzeug 3 fördert, aus dem das Fluid als Fluid-Strahl 25 austritt.

Der Vorratsbehälter 1 und die Pumpe 2, die als Kolben-Zylinder-Einheit ausgebildet ist, sind über eine Saugleitung 4 miteinander verbunden, wobei die Saugleitung 4 mittels einer ersten Kupplung 5 mit dem Vorratsbehälter 1 und mittels einer zweiten Kupplung 6 mit der Pumpe 2 verbunden ist. Die Pumpe 2 ist andererseits mit dem Operationswerkzeug 3 über eine Druckleitung 7 verbunden, wobei die Verbindung mit dem Operationswerkzeug 3 über eine dritte Kupplung 8 realisiert ist. Das Operationswerkzeug 3 umfasst ein Handstück 9 sowie ein Druck- und Saugrohr 10. Das Druck- und Saugrohr 10 besitzt eine innen liegende Druckkanüle mit einer am Ende befindlichen Austrittsöffnung 11, die mit der zum Operationswerkzeug 3 führenden Druckleitung 7 in Verbindung steht. Das Saugrohr 10 ist über eine Absaugleitung 12 sowie eine Absaugpumpe 13 mit einem Aufnahmetank 14 verbunden. Außerdem besitzt das Saugrohr 10 an seinem Umfang verteilt radial verlaufende Absaugöffnungen 15 zur Aufnahme der abgetrennten Segmente und der angesammelten Trennflüssigkeit.

Die Pumpe 2 umfasst einen Zylinder 16, in dem ein Kolben 17 über seinen Kolbenweg 18 translatorisch bewegbar ist. Angetrieben wird der Kolben 17 in seiner translatorischen Bewegung durch einen an ihn angeschlossenen Spindeltrieb 20, dessen Bewegungsenergie wiederum von einer Antriebseinheit 21, vorteilhafterweise einem Elektromotor, zur Verfügung gestellt wird. Auf den konkreten Aufbau des Spindeltriebes soll nicht näher eingegangen werden.

Anstelle des Spindeltriebes kann auch ein linearer Stellzylinder eingesetzt werden, der über eine Steckkupplung mit dem Kolben 17 wirkverbunden ist. Der lineare Stellzylinder umfasst beispielsweise einen Planetenrollengewindetrieb und einen AC-Servomotor. Aufbau und Wirkungsweise sind bekannt, so dass an dieser Stelle hierauf nicht näher eingegangen wird.

Von der Erfindung umfasst ist außerdem eine Steuerungseinrichtung 24, die zumindest mit der Antriebseinheit 21 derart verbunden ist, dass sie deren Drehbewegung beeinflussen kann. Bei Betätigung der Antriebseinheit 21 wird der Spindeltrieb 20, oder der lineare Stellzylinder, derart betätigt, dass der Kolben 17 translatorisch verschoben wird. Bewegt sich der Kolben 17 nach vorne (gemäß Darstellung nach unten), erzeugt er eine Druckerhöhung auf das Fluid, welches sich im Zylinder 16 befindet. Durch die Anordnung eines nicht dargestellten Rückschlagventils in der Saugleitung 4 wird dadurch das Fluid veranlasst, zum Operationswerkzeug 3 zu strömen und aus dem Druck- und Saugrohr 10 als Fluid-Strahl 25 auszutreten.

Wird durch eine Betätigung einer Betätigungseinrichtung 19 am Handstück 9 (oder in nicht dargestellter Weise durch einen Fußschalter) der Fluid-Strahl 25 unterbrochen, wird ein Signal über die Unterbrechung generiert und die Antriebseinheit 21 über die Steuerungseinrichtung 24 derart gesteuert, dass diese ihre Drehrichtung umkehrt und somit über den Spindeltrieb 20, oder den linearen Stellzylinder, den Kolben 17 wieder nach hinten (gemäß Darstellung nach oben) fährt, so dass die Pumpe 2 eine Saugwirkung entfaltet und bewirkt, dass weiteres Fluid aus dem Vorratsbehälter 1 in den Zylinder 16 strömt und somit bei erneuter Umkehrung der Bewegungsrichtung des Kolbens 17 zur Erzeugung des Fluid-Strahles 25 zur Verfügung steht. Die Unterbrechung des Fluid-Strahles 25 kann dabei nicht nur durch ein Signal aufgrund der Betätigung der Betätigungseinrichtung 19 generiert werden, sondern kann alternativ oder zusätzlich durch einen Sensor 22 an der Druckleitung 7 generiert werden oder durch einen Sensor an der Position 23 generiert werden, der feststellt, wann der Kolben 17 in seiner Endposition auf seinem Weg in der Betätigungsweise zur Druckerzeugung gelangt ist.

Auch kann ein Wegmesssystem 26 zur Detektion der Bewegung des Kolbens 17 vorgesehen sein. Dieses Wegmesssystem 26 arbeitet mit dem Spindeltrieb 20 bzw. dem linearen Stellzylinder zusammen.

Es ist ersichtlich, dass trotz des minimalen konstruktiven Aufwandes des Kolbens 17 dieser aufgrund der erfindungsgemäßen Steuerung seiner Bewegung bei Unterbrechung des Fluid-Strahles 25 in der Lage ist, das Zeitfenster der Unterbrechung für eine erneute Befüllung des Zylinders 16 zu nutzen und demzufolge bei Beendigung der Unterbrechung ein größeres Fluid-Volumen zum Strahlen zur Verfügung zu stellen, als es herkömmliche Vorrichtungen vermögen.

Dabei erfolgt die Umschaltung in die Betriebsweise zur Saugwirkungserzeugung automatisch, so dass der Benutzer, insbesondere bei Durchführung einer medizinischen oder chirurgischen Operation, nicht darauf achten muss, erneut für die Befüllung des Zylinders 16 zu sorgen und lediglich zur Beendigung der Unterbrechung des Volumenstroms erneut die Betätigungseinrichtung 19 bedienen muss. Erfolgt die Saugwirkungserzeugung automatisch aufgrund der Ansteuerung des zweiten Sensors/Wegmesssystem 23 , ohne dass der Anwender die Betätigungseinrichtung 19 bedient hat, wird beim Erreichen der hinteren Endposition des Kolbens wiederum automatisch in die Betriebsweise zur Druckerzeugung umgeschaltet. Die erfindungsgemäße Vorrichtung ist somit insbesondere geeignet, als kostengünstiges und leichtes Einweg-gerät zur Fettabsaugung verwendet zu werden.

### Bezugszeichenliste

- 1: Vorratsbehälter
- 2: Pumpe
- 3: Operationswerkzeug
- 4: Saugleitung
- 5: erste Kupplung
- 6: zweite Kupplung
- 7: Druckleitung
- 8: dritte Kupplung
- 9: Handstück
- 10: Druck- und Saugrohr mit innengeführter Druckleitung
- 11: Austrittsöffnung
- 12: Absaugleitung
- 13: Absaugpumpe
- 14: Aufnahmetank
- 15: Absaugöffnung
- 16: Zylinder
- 17: Kolben
- 18: Kolbenweg
- 19: Betätigungseinrichtung
- 20: Spindeltrieb
- 21: Antriebseinheit
- 22: Sensor
- 23: Sensor
- 24: Steuerungseinrichtung
- 25: Fluid-Strahl
- 26: Wegmesssystem

## Patentansprüche

1. Verfahren zur Förderung eines Fluides zwecks Erzeugung eines Fluid-Strahles (25) zum Schneiden und/oder Spülen von festem Material oder weichem Material, bei dem das Fluid von einer Pumpe (2) druckbeaufschlagt mit einem bestimmten Volumenstrom einer Austrittsöffnung (11) zugeleitet wird, **dadurch gekennzeichnet, dass** bei einer Unterbrechung des Volumenstroms die Pumpe (2) automatisch aus einer Betriebsweise zur Druckerzeugung in eine Betriebsweise zur Saugwirkungserzeugung zwecks Ansaugung von Fluid umgeschaltet wird, wobei der Saugbetrieb so lange realisiert wird, bis ein Signal über die Beendigung der Unterbrechung des Volumenstromes generiert wird, woraufhin die Pumpe (2) in die Betriebsweise zur Druckerzeugung umschaltet.

2. Verfahren zur Förderung eines Fluides nach Anspruch 1, **dadurch gekennzeichnet, dass** der Volumenstrom der Austrittsöffnung (11) pulsationsfrei oder pulsierend zugeleitet wird, wobei die Zeitintervalle zwischen den einzelnen, die Pulsierung realisierenden Volumenstrom-Abschnitten zwischen 0,05 und 30 Sekunden betragen.

3. Verfahren zur Förderung eines Fluides nach wenigstens einem der vorhergehenden Ansprüche, **dadurch gekennzeichnet, dass** die Zeitdauer der Betriebsweise zur Druckerzeugung und/oder die Zeitdauer der Betriebsweise zur Saugwirkungserzeugung mittels einer Steuerungseinrichtung (24) vor oder während des Verfahrens eingestellt wird.

4. Verfahren zur Förderung eines Fluides nach Anspruch 3, **dadurch gekennzeichnet, dass** die Zeitdauer für die Betriebsweise zur Saugwirkungserzeugung kürzer, länger oder gleich ist als die Zeitdauer für die Betriebsweise zur Druckerzeugung, abhängig von der Position des Kolbens (17) im Zylinder (16) bei Generierung des Signal zum Umschalten in die Betriebsweise zur Saugwirkungserzeugung.

5. Verfahren zur Förderung eines Fluides nach wenigstens einem der vorhergehenden Ansprüche, **dadurch gekennzeichnet, dass** bei Anwendung einer Kolbenpumpe die Steuerungseinrichtung (24) den Kolbenweg (18) derart steuert, dass der Kolbenweg (18) bei der Betriebsweise zur Saugwirkungserzeugung kürzer, gleich oder länger ist als der in der zuvor realisierten Betriebsweise zur Druckerzeugung zurückgelegte Kolbenweg (18).

6. Vorrichtung zur Erzeugung eines Volumenstromes zwecks Realisierung eines Fluid-Strahles zum Schneiden und/oder Spülen von festem Material oder weichem Material, welche eine Pumpe (2) umfasst, mittels derer eine Betriebsweise zur Druckerzeugung im Fluid und eine Betriebsweise zur Saugwirkungserzeugung realisierbar ist, **dadurch gekennzeichnet, dass** die Vorrichtung derart ausgestaltet ist, dass bei Unterbrechung des eingestellten Volumenstroms die Pumpe (2) automatisch aus der Betriebsweise zur Druckerzeugung in eine Betriebsweise zur Saugwirkungserzeugung zwecks Ansaugung von Fluid umschaltbar ist bzw. umschaltet, wobei der Saugbetrieb so lange realisiert wird, bis ein Signal über die Beendigung der Unterbrechung des Volumenstromes generiert wird, woraufhin die Pumpe (2) in die Betriebsweise zur Druckerzeugung umschaltet, die Vorrichtung eine Betätigungseinrichtung (19) umfasst, mittels derer manuell die Unterbrechung des Volumenstromes realisierbar ist, und die Betätigungseinrichtung (19) derart ausgestaltet ist, dass sie bei Betätigung zwecks Unterbrechung des Volumenstromes ein Signal über die Unterbrechung des Volumenstromes generiert, welches vorzugsweise an eine Antriebseinheit (21) mit Steuerungseinrichtung (24) weitergeleitet wird.

7. Vorrichtung zur Erzeugung eines Volumenstromes nach Anspruch 6 **dadurch gekennzeichnet, dass** die Pumpe (2) eine Kolbenpumpe mit nur einer Kolben-Zylinder-Einheit ist, die vorzugsweise über nur ein Rückschlagventil in der Saugleitung verfügt.

8. Vorrichtung zur Erzeugung eines Volumenstromes nach Anspruch 7 **dadurch gekennzeichnet, dass** die Vorrichtung einen motorisch betriebenen Spindeltrieb (20), oder einen linearen Stellzylinder, umfasst und der Kolben (17) mittels des motorisch betriebenen Spindeltriebes (20), oder des linearen Stellzylinders, bewegbar ist.

9. Vorrichtung zur Erzeugung eines Volumenstromes nach wenigstens einem der Ansprüche 7 oder 8, **dadurch gekennzeichnet, dass** die Kolbenpumpe motorisch angetrieben ist und die Vorrichtung ein Getriebe umfasst, welches bei gleicher Drehzahl des motorischen Antriebes des Kolbens (17) eine Bewegung des Kolbens (17) in der Betriebsweise zur Saugwirkungserzeugung mit größerer Geschwindigkeit als die Bewegung des Kolbens (17) in der Betriebsweise zur Druckerzeugung realisierbar macht.

10. Vorrichtung zur Erzeugung eines Volumenstromes nach wenigstens einem der Ansprüche 7 oder 8, **dadurch gekennzeichnet, dass** die Kolbenpumpe motorisch angetrieben ist und der motorische Antrieb in der Betriebsweise der Saugwirkungserzeugung, wo nur eine geringe Last wirkt, eine entsprechend höhere Drehzahl generiert als in der Betriebsweise der Druckerzeugung, wo eine entsprechend höhere Last wirkt.

11. Vorrichtung zur Erzeugung eines Volumenstromes nach wenigstens einem der Ansprüche 6 bis 10, **dadurch gekennzeichnet, dass** die Vorrichtung einen Sensor (22) an einer in Volumenstromrichtung der Pumpe (2) nachgeschalteten Leitung aufweist, mittels dem eine Unterbrechung des Volumenstromes erfassbar ist.

12. Vorrichtung zur Erzeugung eines Volumenstromes nach wenigstens einem der Ansprüche 6 bis 11, **dadurch gekennzeichnet, dass** die Vorrichtung an der Antriebseinheit (21) mit Spindeltrieb oder der lineare Stellzylinder ein Wegmesssystem (26) aufweist, mittels dem die Positionierung des Kolbens (17) in einer Endposition bei der Betriebsweise zur Druckerzeugung und damit die Unterbrechung des Volumenstromes detektierbar ist.

13. Vorrichtung zur Erzeugung eines Volumenstromes nach wenigstens einem der Ansprüche 6 bis 12, **dadurch gekennzeichnet, dass** die Vorrichtung eine Steuerungseinrichtung (24) umfasst, die derart ausgestaltet ist, dass mit ihr die zu erzeugenden Volumenstrom-Intervalle und/oder die Zeitabstände zwischen den Volumenstrom-Intervallen und/oder die Zeitdauern für die beiden Betriebsweisen zum Ansaugen und Druckerzeugen einstellbar sind.

## Claims

1. A method for conveying a fluid for the purpose of generating a fluid jet (25) for cutting and/or flushing of solid material or soft material, in which the fluid is delivered from a pump (2) under pressure with a determined volume flow to an exit opening (11), **characterized in that** in the event of an interruption of the volume flow, the pump (2) is automatically switched from an operating mode for generating pressure to an operating mode for generating suction for the purpose of suctioning fluid, wherein the suction operation is realized until a signal about the termination of the interruption of the volume flow is generated, whereafter the pump (2) is switched over to the operating mode for generating pressure.

2. The method for conveying a fluid according to Claim 1, **characterized in that** the volume flow is delivered to the exit opening (11) without pulsation or pulsating, wherein the time intervals between the individual volume flow segments realizing the pulsation are between 0.05 and 30 seconds.

3. The method for conveying a fluid according to at least one of the preceding claims, **characterized in that** the time duration of the operating mode for generating pressure and/or the time duration of the operating mode for generating suction is set with a control apparatus (24) before or during the process.

4. The method for conveying a fluid according to Claim 3, **characterized in that** the time duration of the operating mode for generating suction is shorter or longer than or identical to the time duration for the operating mode for generating pressure, depending on the position of the piston (17) in the cylinder (16) at the time the signal for switching into the operating mode for generating suction is generated.

5. The method for conveying a fluid according to at least one of the preceding claims, **characterized in that** when using a piston pump, the control apparatus (24) controls the piston stroke (18) such that the piston stroke (18) during the operating mode for generating suction is shorter than, identical to or longer than the piston stroke (18) performed in the previously realized operating mode for generating pressure.

6. A device for producing a volume flow for realizing a fluid jet for cutting and/or flushing of solid material or soft material, comprising a pump (2) with which an operating mode for generating pressure in the fluid and an operating mode for generating suction can be realized, **characterized in that** the device is configured such that during interruption of the set volume flow, the pump (2) can be switched over or switches over automatically from the operating mode for generating pressure to an operating mode for generating suction for the purpose of suctioning fluid, wherein the suction operation is realized until a signal about the termination of the interruption of the volume flow is generated, whereafter the pump (2) is switched over to the operating mode for generating pressure, the device comprises an operating apparatus (19) by means of which the volume flow can be interrupted manually, and the operating apparatus (19) is configured so as to generate upon operation for interrupting the volume flow a signal of the interruption of the volume flow, which signal is preferably transmitted to a drive unit (21) having a control apparatus (24).

7. The device for producing a volume flow according to Claim 6, **characterized in that** the pump (2) is a piston pump with a single piston-cylinder unit, which preferably has only a single check valve in the suction line.

8. The device for producing a volume flow according to Claim 7, **characterized in that** the device comprises a motor-driven spindle drive (20) or a linear actuator cylinder, and the piston (17) is movable with the motor-driven spindle drive (20) or the linear actuator cylinder.

9. The device for producing a volume flow according to at least one of Claims 7 or 8, **characterized in that** the piston pump is motor-driven and the device comprises a gear, which makes it possible to realize with identical rotation speed of the motor drive of the piston (17) a movement of the piston (17) in the operating mode for generating suction with greater speed than the movement of the piston (17) in the operating mode for generating pressure.

10. The device for producing a volume flow according to at least one of Claims 7 or 8, **characterized in that** the piston pump is motor-driven and the motor drive in the operating mode for generating suction, when only a small load is applied, generates a correspondingly higher rotation speed than in the operating mode for generating pressure where a correspondingly higher load is applied.

11. The device for producing a volume flow according to at least one of Claims 6 to 10, **characterized in that** the device comprises a sensor (22) on a line disposed downstream of the pump (2) in the direction of the volume flow, by means of which an interruption of the volume flow can be captured.

12. The device for producing a volume flow according to at least one of Claims 6 to 11, **characterized in that** the device comprises a distance measuring system (26) disposed on the drive unit (21) with spindle drive or on the linear actuator cylinder, by means of which distance measuring system the positioning of the piston (17) in an end position during the operating mode for generating pressure and hence the interruption of the volume flow can be detected.

13. The device for producing a volume flow according to at least one of Claims 6 to 12, **characterized in that** the device comprises a control apparatus (24) which is configured so as to be able to set the volume flow intervals to be generated by the control apparatus (24) and/or the time intervals between the volume flow intervals and/or the time durations for the two operating modes for generating suction and pressure.

## Revendications

1. Procédé pour transporter un fluide dans le but de produire un jet de fluide (25) destiné à couper et/ou rincer un matériau solide ou un matériau tendre, dans lequel le fluide est alimenté pressurisé par une pompe (2) dans un orifice de sortie (11) avec un débit volumétrique précis, **caractérisé en ce que,** lors d'une interruption du débit volumétrique, la pompe (2) est commutée automatiquement d'un mode de fonctionnement pour la production de pression à un mode de fonctionnement pour la production d'un effet d'aspiration dans le but d'aspirer du fluide, le fonctionnement en aspiration étant réalisé jusqu'à ce qu'un signal indiquant la fin de l'interruption du débit volumétrique soit généré, après quoi la pompe (2) commute dans le mode de fonctionnement pour la production de pression.

2. Procédé pour transporter un fluide selon la revendication 1, **caractérisé en ce que** le débit volumétrique est alimenté dans l'orifice de sortie (11) sans pulsation ou de manière pulsatoire, les intervalles de temps entre chaque période de débit volumétrique réalisant la pulsation durant entre 0,05 et 30 secondes.

3. Procédé pour transporter un fluide selon au moins une des revendications précédentes, **caractérisé en ce que** la durée du mode de fonctionnement pour la production de pression et/ou la durée du mode de fonctionnement pour la production d'un effet d'aspiration est réglée avant ou pendant le procédé au moyen d'un appareil de commande (24).

4. Procédé pour transporter un fluide selon la revendication 3, **caractérisé en ce que** la durée pour le mode de fonctionnement pour la production d'un effet d'aspiration est inférieure, supérieure ou égale à la durée pour le mode de fonctionnement pour la production de pression, en fonction de la position du piston (17) dans le cylindre (16) lors de la génération du signal destiné à la commutation dans le mode de fonctionnement pour la production d'un effet d'aspiration.

5. Procédé pour transporter un fluide selon au moins une des revendications précédentes, **caractérisé en ce que,** lors de l'utilisation d'une pompe à piston, l'appareil de commande (24) commande la course du piston (18) de telle manière que, en mode de fonctionnement pour la production d'un effet d'aspiration, la course de piston (18) est plus courte, identique ou plus longue que la course de piston (18) parcourue dans le mode de fonctionnement pour la production de pression réalisé auparavant.

6. Dispositif pour produire un débit volumétrique dans le but de réaliser un jet de fluide destiné à couper et/ou rincer un matériau solide ou un matériau tendre, qui comprend une pompe (2), au moyen de laquelle un mode de fonctionnement pour la production de pression dans le fluide et un mode de fonctionnement pour la production d'un effet d'aspiration peuvent être réalisés, **caractérisé en ce que** le dispositif est conçu de telle manière que, lors d'une interruption du débit volumétrique réglé, la pompe (2) commute ou peut être commutée automatiquement d'un mode de fonctionnement pour la production de pression à un mode de fonctionnement pour la production d'un effet d'aspiration dans le but d'aspirer du fluide, le fonctionnement en aspiration étant réalisé jusqu'à ce qu'un signal indiquant la fin de l'interruption du débit volumétrique soit généré, après quoi la pompe (2) commute dans le mode de fonctionnement pour la production de pression, le dispositif comprenant un appareil d'actionnement (19), au moyen duquel l'interruption du débit volumétrique peut être réalisée manuellement, et l'appareil d'actionnement (19) étant conçu de telle manière que, lors de l'actionnement dans le but d'interrompre le débit volumétrique, il génère un signal indiquant l'interruption du débit volumétrique, qui est de préférence transféré à une unité d'entraînement (21) dotée d'un appareil de commande (24).

7. Dispositif pour produire un débit volumétrique selon la revendication 6, **caractérisé en ce que** la pompe (2) est une pompe à piston comprenant une seule unité piston-cylindre, qui dispose de préférence d'une seule soupape de non-retour dans la conduite d'aspiration.

8. Dispositif pour produire un débit volumétrique selon la revendication 7, **caractérisé en ce que** le dispositif comprend une commande à vis (20) entraînée par moteur, ou un cylindre de commande linéaire, et le piston (17) peut être déplacé au moyen de la commande à vis (20) entraînée par moteur ou du cylindre de commande linéaire.

9. Dispositif pour produire un débit volumétrique selon au moins une des revendications 7 ou 8, **caractérisé en ce que** la pompe à piston est entraînée par moteur et le dispositif comprend un mécanisme de transmission qui, à vitesse de rotation identique de l'entraînement par moteur du piston (17), permet de réaliser un déplacement du piston (17) dans le mode de fonctionnement pour la production d'un effet d'aspiration à une vitesse supérieure au déplacement du piston (17) dans le mode de fonctionnement pour la production de pression.

10. Dispositif pour produire un débit volumétrique selon au moins une des revendications 7 ou 8, **caractérisé en ce que** la pompe à piston est entraînée par moteur et, dans le mode de fonctionnement de production d'un effet d'aspiration, dans lequel seulement une charge faible agit, l'entraînement par moteur génère une vitesse de rotation corrélativement supérieure par rapport au mode de fonctionnement de production de pression, dans lequel une charge corrélativement supérieure agit.

11. Dispositif pour produire un débit volumétrique selon au moins une des revendications 6 à 10, **caractérisé en ce que** le dispositif comporte un capteur (22) sur une conduite placée en aval dans le sens du débit volumétrique de la pompe (2), au moyen duquel une interruption du débit volumétrique peut être détectée.

12. Dispositif pour produire un débit volumétrique selon au moins une des revendications 6 à 11, **caractérisé en ce que** le dispositif comporte, sur l'unité d'entraînement (21) dotée d'une commande à vis ou sur le cylindre de commande linéaire, un système de mesure de déplacement (26), au moyen duquel le positionnement du piston (17) dans une position finale en mode de fonctionnement pour la production de pression, et ainsi l'interruption du débit volumétrique, peut être détecté.

13. Dispositif pour produire un débit volumétrique selon au moins une des revendications 6 à 12, **caractérisé en ce que** le dispositif comprend un appareil de commande (24), qui est conçu de telle manière qu'au moyen de celui-ci, les intervalles de débit volumétrique à produire et/ou les laps de temps entre les intervalles de débit volumétrique et/ou les durées pour les deux modes de fonctionnement pour l'aspiration et la production de pression peuvent être réglés.
